# EUROPÄISCHE PATENTSCHRIFT

(11) **EP 0 538 249 B1**
(45) Veröffentlichungstag und Bekanntmachung des Hinweises auf die Patenterteilung: **21.09.1994**
(21) Anmeldenummer: 90910720.3
(22) Anmeldetag: 07.07.1990
(51) Int. Cl.: A61F 9/00

(54) **VERFAHREN UND VORRICHTUNG ZUR MODELLIERUNG ODER KORREKTUR VON OPTISCHEN LINSEN, INSBESONDERE DER HORNHAUT DES AUGES**
PROCESS AND DEVICE FOR MODELLING OR CORRECTING OPTICAL LENSES, ESPECIALLY THE CORNEA OF THE EYE
PROCEDE ET DISPOSITIF DE MODELAGE OU DE CORRECTION DE LENTILLES OPTIQUES, NOTAMMENT DE LA CORNEE DE L'OEIL

(43) Veröffentlichungstag der Anmeldung: 28.04.1993
(73) Patentinhaber: AESCULAP AG, D-78501 Tuttlingen (DE)
(72) Erfinder: SCHRÖDER, Eckhard, D-8501 Eckental (DE); FIEDLER, Joachim, D-7180 Crailsheim (DE); PIEGER, Stefan, D-8500 Nürnberg (DE)
(74) Vertreter: Hoeger, Stellrecht & Partner
(86) Internationale Anmeldenummer: EP9001101
(87) Internationale Veröffentlichungsnummer: WO9200711

(56) Entgegenhaltungen:
- EP-A- 0 296 982
- EP-A- 0 346 116
- EP-A- 0 402 250
- DE-A- 3 535 073
- DE-A- 3 615 042
- DE-A- 3 922 819
- US-A- 4 718 418
- US-A- 4 732 148

## Beschreibung

Die Erfindung betrifft ein Verfahren zur Modellierung oder Korrektur von optischen Linsen mit den im Oberbegriff des Patentanspruches 1 angegebenen Merkmalen. Außerdem betrifft die Erfindung eine Vorrichtung zur Modellierung oder Korrektur von optischen Linsen, insbesondere der Hornhaut des Auges, mit den im Oberbegriff des Patentanspruches 2 angegebenen Merkmalen.

Eine solche Methode zur Korrektur der Brechkraft der Augenhornhaut oder von optischen Linsen, beispielsweise bei Kontaktlinsen, trägt das Linsenmaterial in konzentrischen Kreisringflächen unterschiedlich tief ab, so daß auf diese weise eine gewünschte Querschnittsform der Linse erreicht werden kann. Üblicherweise ist die Zahl der konzentrischen Linsenbereiche, die unterschiedlich tief abgetragen werden, sehr groß, das heißt sehr schmale Kreisringflächen schließen unmittelbar aneinander an (US-A-4 718 418; DE-A-36 15 042).

In der Praxis ist es außerordentlich schwierig, mit der erforderlichen Präzision eine große Anzahl von konzentrischen Kreisringflächen, zum Beispiel in der Größenordnung von 100, exakt mit der gewünschten Zahl von Laserimpulsen zu bestrahlen, um die gewünschte Abtragungsrate in jedem einzelnen Kreisringbereich zu erzielen. Es ist zu diesem Zweck bekannt, nacheinander verschiedene Blenden vor die zu modellierende Linsenoberfläche zu setzen, wobei jede Blende andere Kreisringflächen für die Laserstrahlung freigibt. Die freigegebenen Kreisringflächen müssen genau konzentrisch zueinander angeordnet sein und dürfen sich weder überlappen, noch dürfen zwischen benachbarten Kreisringflächen unbelichtete Spalte verbleiben. Bei der Lösung dieser Aufgabe haben sich in der Praxis erhebliche Probleme ergeben.

Bei einem Verfahren der eingangs beschriebenen Art wird diese Aufgabe erfindungsgemäß durch die kennzeichnenden Merkmale des Patentanspruches 1, bei einer Vorrichtung der eingangs beschriebenen Art durch die kennzeichnenden Merkmale des Patentanspruches 2 gelöst.

Es wird also nur eine einzige Maske verwendet, die in Umfangsrichtung unterschiedlich lange Ausnehmungen aufweist, beispielsweise Spalte. Der Verlauf jeder einzelnen Ausnehmung entspricht einer Kreisringfläche um die Drehachse der Maske, die Länge dieser Ausnehmung ist jedoch kürzer als die gesamte Kreisringfläche, wobei die einzelnen Ausnehmungen in Umfangsrichtung verschiedene Erstreckung haben. Durch die schrittweise Weiterdrehung dieser Maske wird trotzdem die jeder Ausnehmung zugeordnete Kreisringfläche vollständig von Laserstrahlungsimpulsen getroffen, wobei bei in Umfangsrichtung kurzen Ausnehmungen nur einer oder wenige Impulse die darunterliegende Linsenoberfläche treffen, bei langen Ausnehmungen dagegen viele. Auf diese Weise kann die Abtragungsrate präzise kontrolliert werden, außerdem besteht nicht die Gefahr, daß in radialer Richtung Überlappungen mit Doppelbelichtungen oder unbelichtete Zonen verbleiben, da die Ausnehmungen selbstverständlich so auf der Maske angeordnet werden können, daß sie in radialer Richtung präzise unmittelbar aneinander anschließen, beispielsweise dadurch, daß mehrere Einzelausnehmungen zusammen eine Durchbrechung in der Maske bilden, deren Randkontur entsprechend der unterschiedlichen Länge der einzelnen Ausnehmungen abgestuft ist.

Besonders vorteilhaft ist es, wenn die Länge der Ausnehmungen in der Maske ein ganzzahliges Vielfaches der Schrittweite der Maskendrehung ist. Dadurch wird sichergestellt, daß die Bestrahlungsrate in unterschiedlichen Kreisringzonen um eine genau definierte Impulszahl unterschiedlich ist, wobei die Impulszahldifferenz aus der Anzahl der Impulse resultiert, die in einer Stillstandsphase der Maske auf die zu modellierende Linsenoberfläche auftreffen.

Normalerweise wird eine Maske für eine Behandlung um 360° gedreht, so daß sichergestellt ist, daß in jeder Kreisringzone eine vollständige Abtragung erfolgt ist. Es ist aber auch möglich, die Maske punktsymmetrisch bezüglich der Drehachse auszubilden, dann genügt pro Behandlung eine Drehung um 180°.

Um während der einzelnen Impulsdauern eine gleichmäßige Bestrahlung der gesamten von den Ausnehmungen freigegebenen Fläche zu gewährleisten, wobei trotz beschränkter Laserenergie die pro Flächeneinheit auf die Linsenoberfläche auftreffende Energie zur Abtragung ausreichend sein muß, kann es vorteilhaft sein, wenn der Laserimpuls durch die optischen Abbildungsmittel auf eine balkenförmige Fläche abgebildet wird und wenn durch weitere optische Elemente die balkenförmige Strahlung senkrecht zur Längsausdehnung der balkenförmigen Fläche derart abgelenkt wird, daß die balkenförmige Strahlung die Maske bei jedem Impuls vollständig überstreicht. Durch diese Maßnahme wird die zur Verfügung stehende Laserstrahlung in einen begrenzten Bereich konzentriert, so daß die Energiedichte relativ hoch ist. Durch Überstreichen der Fläche mittels des Balkenbereiches wird trotzdem die gesamte zu behandelnde Fläche bei jedem Impuls gleichmäßig beaufschlagt. Durch diese Maßnahme kann einerseits eine relativ hohe Energiedichte erreicht werden, andererseits wird diese Energie sehr gleichmäßig auf die Fläche verteilt, denn im Gegensatz zum zeilenförmigen Abrastern einer Fläche ergeben sich bei dieser Maßnahme keinerlei Probleme einer ungleichmäßigen Ausleuchtung durch nicht exakt aneinander anschließende Zeilen.

Die Maske ist vorteilhafterweise an einer starr mit der Linse verbundenen Halterung gelagert, die vorzugsweise mit Hilfe einer Unterdruckkammer an der Oberfläche der Linse festgesaugt ist. Die Maske kann an der Halterung mittels eines Kugellagers gelagert sein.

Besonders vorteilhaft ist es, wenn die Umfangslänge der Ausnehmungen im radial außenliegenden Bereich der Maske von innen nach außen abnimmt. Dadurch ist sichergestellt, daß im Außenbereich der behandelten Oberfläche eine geringe Abtragung erfolgt, so daß dort kein stufenförmiger Übergang zwischen dem behandelten Bereich und dem umgebenden, nicht behandelten Bereich auftritt. Besonders vorteilhaft ist es dabei, wenn die Umfangslänge der äußersten Ausnehmung eine oder maximal einige wenige Schrittweiten des Maskenantriebs beträgt. Man erhält auf diese Weise einen quasi stetigen Übergang von der behandelten Fläche zur umgebenden nicht behandelten Fläche.

Die nachfolgende Beschreibung bevorzugter Ausführungsformen der Erfindung dient im Zusammenhang mit der Zeichnung der näheren Erläuterung. Es zeigen:
- Figur 1:: eine schematische Seitenansicht einer Behandlungsvorrichtung zur Modellierung oder Korrektur der Augenhornhaut;
- Figur 2:: eine Draufsicht auf ein sehr vereinfachtes Beispiel einer Maske mit in Umfangsrichtung unterschiedlich langen Ausnehmungen;
- Figur 3:: eine Ansicht ähnlich Figur 2, wobei die Maske um eine Schrittweite gedreht ist;
- Figur 4:: eine Draufsicht auf eine punktsymmetrische Maske;
- Figur 5:: eine schematische Querschnittsansicht einer Hornhaut mit einer Korrektur bei Kurzsichtigkeit;
- Figur 6:: eine Ansicht ähnlich Figur 5 bei einer Korrektur der Weitsichtigkeit und
- Figur 7:: eine Draufsicht auf ein weiteres Ausführungsbeispiel einer Maske.

Die Vorrichtung zur Korrektur oder Modellierung einer Linse wird im folgenden am Beispiel der Korrektur der Augenhornhaut beschrieben; es versteht sich jedoch, daß das erfindungsgemäße Verfahren und diese Vorrichtung zur Korrektur andersgearteter Linsen eingesetzt werden können, beispielsweise bei Kontaktlinsen, die aus Material bestehen, das durch Laserstrahlung abgetragen werden kann.

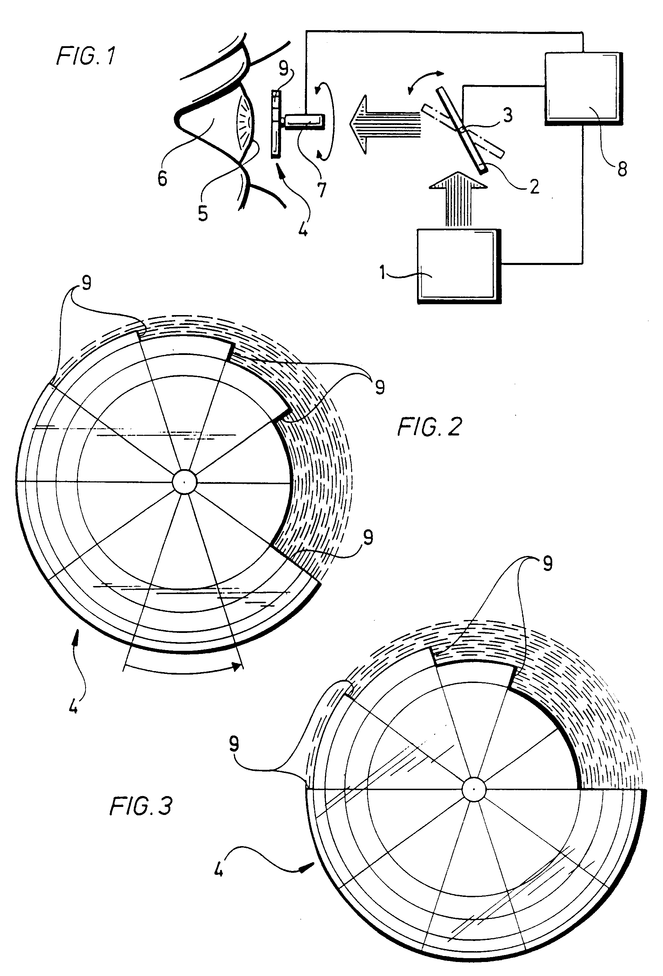

In Figur 1 ist die verwendetete Vorrichtung sehr schematisch dargestellt. Ein Laser 1, beispielsweise ein Excimerlaser, erzeugt Strahlungsimpulse einer bestimmten Dauer, die in Figur 1 durch einen Pfeil symbolisiert werden. In der Zeichnung nicht dargestellte optische Mittel, beispielsweise Zylinderlinsen, fokussieren die Strahlung auf einen balkenförmigen Bereich, der auf einen Schwenkspiegel 2 auffällt, dessen Schwenkachse 3 parallel zu dem balkenförmigen Strahlungsfeld gerichtet ist. Bei jedem Strahlungsimpuls wird der Schwenkspiegel 2 in einem bestimmten Winkelbereich kontinuierlich verschwenkt, so daß der balkenförmige Strahlungsbereich die gesamte Fläche einer Maske 4 überstreicht, die vor der zu behandelnden Hornhaut 5 angeordnet ist. Dazu ist auf den Augapfel 6 eine den zu behandelnden Bereich der Hornhaut 5 umgebende, in der Zeichnung nicht dargestellte Saugmanschette aufgesaugt, die als Halterung für die Maske 4 dient. Die Maske ist dabei mittels eines Kugellagers drehbar an der Halterung gelagert, wobei die Drehachse der Maske 4 genau mit der Sehachse des Auges übereinstimmt. Eine geeignete Justierung läßt sich mit Hilfe eines Pilotstrahles in an sich bekannter Weise erzielen.

Die Maske 4, deren genauere Ausgestaltung weiter unten an Hand der übrigen Figuren näher erläutert wird, ist mit einem Drehantrieb 7 gekoppelt, der die Maske 4 schrittweise dreht und nach jedem Drehschritt in der erreichten Position fixiert. Die Drehwinkel sind dabei so gewählt, daß nach einer bestimmten Anzahl von Schritten die Maske um genau 180° beziehungszweise 360° gedreht worden ist.

Eine Steuerung 8 koordiniert das schrittweise Verdrehen der Maske 4, die Schwenkbewegung des Schwenkspiegels 2 und die Zündung des Lasers 1 in der Weise, daß eine Zündung des Lasers mit entsprechender Schwenkbewegung des Schwenkspiegels immer nur beim Stillstand der Maske 4 erfolgt. Die Zahl der Impulse, die dann pro Stillstandsphase der Maske auf diese gerichtet werden, ist für jede Stillstandsphase gleich, bei entsprechend kleiner Schrittweite der Maskendrehung wird in der Stillstandsphase nur eine Zündung erfolgen, bei entsprechend größeren Schrittweiten oder bei Fällen, in denen eine starke Abtragung erforderlich ist, können auch mehrere Impulse pro Stillstandsphase erzeugt werden.

Der Aufbau der Maske ergibt sich zunächst aus den Darstellungen der Figuren 2 und 3, wobei diese Darstellungen zur Erläuterung der Funktionsweise stark vereinfacht sind.

Die Maske besteht aus einem für die Laserstrahlung undurchdringlichen Material und weist konzentrisch zur Drehachse mehrere in Umfangsrichtung verlaufende Ausnehmungen 9 auf, deren Umfangslänge unterschiedlich ist. Die Ausnehmungen 9 sind dabei so angeordnet, daß sie sich in radialer Richtung unmittelbar aneinander anschließen, wobei dies - wie in der Darstellung der Figuren 2 und 3 gezeigt - dazu führt, daß mehrere Ausnehmungen gemeinsam einen durchbrochenen Bereich bilden. Die Ausnehmungen können aber auch in Umfangsrichtung gegeneinander versetzt sein, so daß zwar in radialer Richtung ein unmittelbarer Anschluß benachbarter Ausnehmungen gewährleistet ist, daß diese aber nicht räumlich unmittelbar nebeneinander liegen, so daß unter Umständen eine Fertigung der Maske erleichtert werden kann.

Unter "Ausnehmung" wird dabei jede Ausbildung der Maske verstanden, die diese für die Laserstrahlung durchlässig gestaltet. Diese Ausnehmung kann einfach eine Durchbrechung der Maske sein, es kann sich aber im Bereich der Ausnehmungen auch Material befinden, das für die Laserstrahlung durchlässig ist, so daß dieses Material zusammen mit dem Material der Maske eine stabile Baueinheit bildet. Dies kann aus Gründen der Stabilität vorteilhaft sein.

Die Umfangslängen der Ausnehmungen 9 unterscheiden sich voneinander um ganzzahlige Vielfache der Schrittweite des Drehantriebes 7. Bei dem Ausführungsbeispiel der Figur 2 beträgt die Länge der innersten Ausnehmung 9 zwei Schrittweiten, jede daran nach außen hin anschließende Ausnehmung ist um eine Schrittweite länger als die jeweils an der Innenseite anschließende.

Durch diese Ausbildung der Maske befindet sich in jeder Kreisringzone der Hornhaut 5, die von einer Ausnehmung 9 der Maske 4 abgedeckt wird, zu jedem Zeitpunkt ein bestimmter Sektor unterhalb der jeweiligen Ausnehmung 9, während der übrige Sektorenbereich der Kreisringzone von der Maske abgedeckt wird. Im Bereich der innersten Ausnehmung der Maske in den Figuren 2 und 3 wird jeder Sektor der Kreisringzone der Hornhaut mit einer Länge von einer Schrittweite zweimal für die Strahlungsimpulse freigegeben, da die Umfangslänge der innersten Ausnehmung zwei Schrittweiten beträgt. Weiter außen liegende Kreisringzonen werden entsprechend der größeren Länge dieser Ausnehmungen häufiger der Laserstrahlung ausgesetzt, jeder Kreisringzonensektor mit einer Länge von 1 Schrittweite wird dabei n-mal bestrahlt, wobei n die Anzahl der Winkelschritte angibt, die der jeweiligen Länge der zugehörigen Ausnehmung entsprechen.

Durch geeignete Ausgestaltung der Maske ergibt sich somit die Möglichkeit, für jede Kreisringzone individuell die gewünschte Anzahl von Laserimpulsen zu bestimmen, denen diese Kreisringzone ausgesetzt wird. Dies erfolgt einfach durch geeignete Wahl der Länge der entsprechenden Ausnehmung der Maske.

Bei der in den Figuren 2 und 3 dargestellten Maske wird diese so oft gedreht, bis die Ausgangsposition wieder erreicht wird, also ist für eine Behandlung eine volle Umdrehung notwendig. Es schließen sich dann die behandelten Bereiche einer Kreisringzone über den gesamten Kreis aneinander an.

In der Praxis wird die Zahl der Ausnehmungen einer Maske wesentlich größer gewählt als dies in den Figuren 2 und 3 dargestellt ist, außerdem kann auch die Differenz der Länge benachbarter Ausnehmungen verschieden sein, dies hängt von der gewünschten Korrektur der Hornhaut ab.

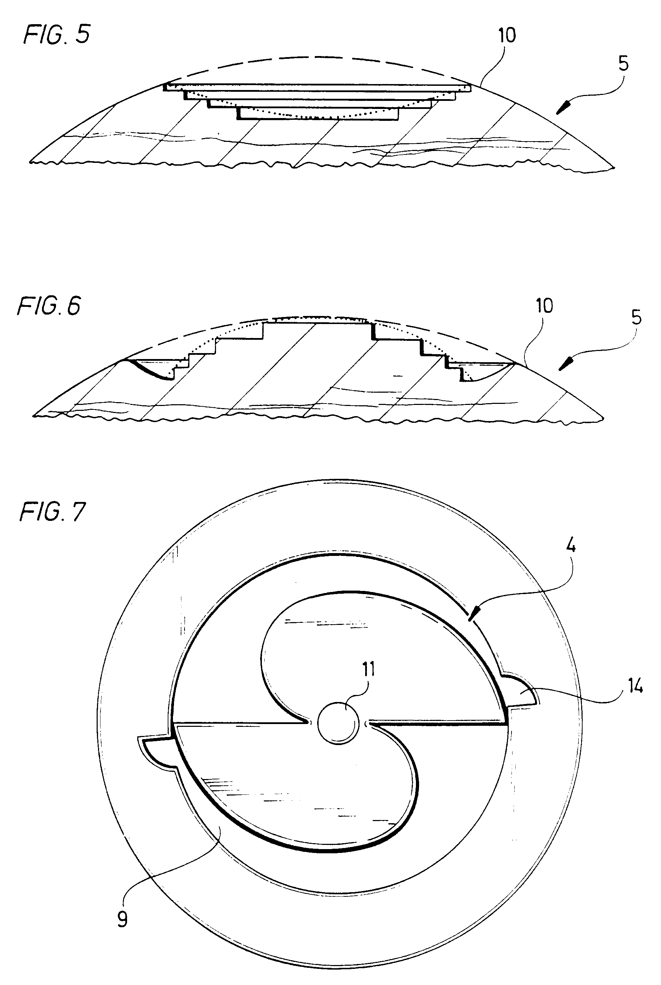

In den Figuren 5 und 6 sind schematisch Querschnitte von derartigen Korrekturen dargestellt. Zur Korrektur der Kurzsichtigkeit (Figur 5) ist es notwendig, in einem zentralen Bereich eine starke Abtragung zu erzielen, die nach außen hin abnimmt. Dementsprechend werden die Ausnehmungen 9 in der Maske im inneren Bereich so gewählt, daß eine große Zahl von Impulsen auf die Hornhaut trifft, diese Zahl nimmt nach außen hin ab.

Bei der Korrektur der Weitsichtigkeit (Figur 6) wird umgekehrt vorgegangen, hier werden im inneren Bereich der Maske nur kurze Ausnehmungen 9 vorgesehen, deren Länge nach außen hin zunimmt. Dadurch erfolgt eine verstärkte Abtragung zum Außenbereich hin. Um hier jedoch einen scharfkantigen Übergang mit dem anschließenden, unbehandelten Bereich 10 der Hornhaut zu vermeiden, ist es günstig, wenn die Länge der Ausnehmungen im Außenbereich auch bei der Weitsichtigkeitskorrektur abnehmend ausgestaltet wird, vorzugsweise so, daß ein stetiger Übergang in den unbehandelten Bereich 10 erreicht werden kann.

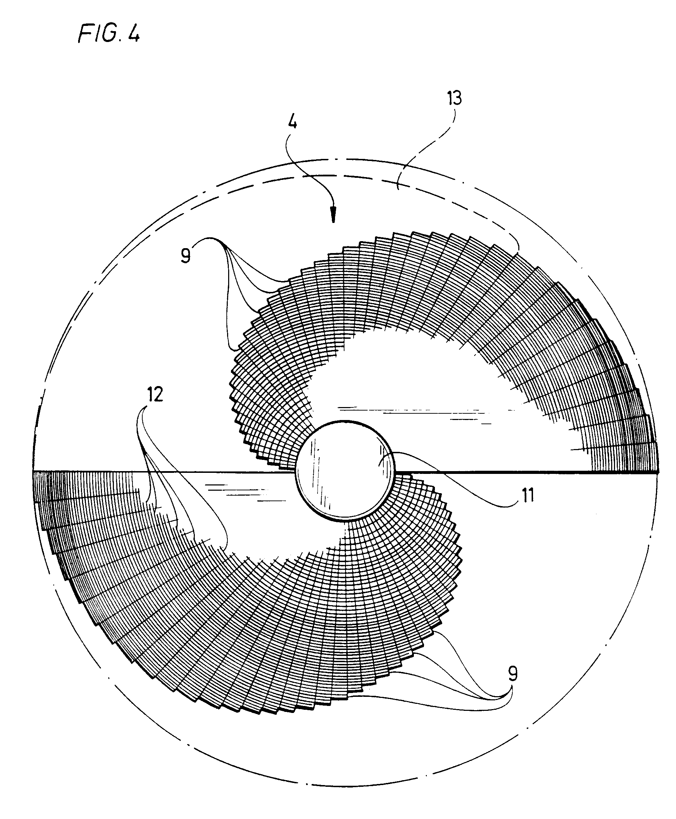

Bei einem bevorzugten Ausführungsbeispiel wird die Maske punktsymmetrisch aufgebaut, so daß bereits bei einer Drehung um 180° eine vollständige Behandlung jeder Kreisringzone erfolgt ist. Das in Figur 4 dargestellte Beispiel ist für eine Korrektur der Weitsichtigkeit bestimmt, dementsprechend ist ein zentraler, vollständig undurchlässiger Bereich 11 vorgesehen, an den sich nach außen hin länger werdende Ausnehmungen 9 anschließen. Zur Verdeutlichung ist in dieser Darstellung auf der Maske, also auf dem undurchlässigen Teil, der Verlauf der konzentrischen Kreisringzonen 12 angedeutet. Diese verbleibenden Maskenteile haben jeweils die Form einer Herzhälfte, wobei diese beiden Teile punktsymmetrisch zueinander angeordnet sind.

In der linken Hälfte der Figur 4 ist strichpunktiert eine mögliche Modifikation angedeutet, bei welcher die Länge der Ausnehmungen im radial außen liegenden Bereich geringer ist, das heißt die Maske weist in diesem Bereich einen die Randzonen abdeckenden Vorsprung 13 auf. Mit dieser Ausgestaltung gelingt es, den oben beschriebenen und in Figur 6 dargestellten stetigen Übergang zu dem unbehandelten Bereich 10 der Hornhaut zu erzielen.

Eine Maske zur Behandlung der Kurzsichtigkeit hat eine ähnliche Randkontur wie dies in Figur 4 dargestellt ist, nur ist in diesem Falle der herzförmige Bereich für den Laser durchlässig, während der übrige Bereich von der Maske abgedeckt wird. In diesem Falle ist der Vorsprung 13 nicht notwendig, da die Länge der Ausnehmungen 9 im radial außen liegenden Bereich ohnehin laufend abnimmt.

Es wäre im übrigen auch möglich, statt des Vorsprunges 13 die in Figur 4 dargestellten Maskenbereiche noch zu umgeben mit einem Maskenbereich, der zusätzlich mehrere Ausnehmungen aufweist, deren Länge von innen nach außen hin abnimmt. Ein Beispiel einer solchen Maske ist in Figur 7 dargestellt, dabei erkennt man viertelkreisförmige Durchbrechungen 14 im Außenbereich der Maske, die den kontinuierlichen Übergang vom behandelten Bereich der Hornhaut in den unbenandelten Bereich 10 gewährleisten.

## Patentansprüche

1. Verfahren zur Modellierung oder Korrektur von optischen Linsen mit einem Impulslaser, dessen Strahlungsimpulse über optische Abbildungsmittel und Blenden derart auf die zu modellierende Oberfläche der Linse gerichtet werden, daß konzentrische Kreisringflächen auf der Linsenoberfläche mit einer unterschiedlichen Zahl von Strahlungsimpulsen beaufschlagt und daher unterschiedlich tief abgetragen werden, wobei man die zu behandelnde Linsenoberfläche mittels einer um eine linsenfeste, senkrecht auf der Linsenoberfläche stehende Achse drehbaren Maske teilweise abdeckt,
dadurch gekennzeichnet, daß man eine Maske verwendet, die in radialer Richtung mehrere in Umfangsrichtung und konzentrisch zueinander verlaufende Ausnehmungen aufweist, deren Länge in Umfangsrichtung verschieden ist, daß man die Maske schrittweise verdreht und daß man den Laser jeweils nach der Weiterdrehung der Maske während des Stillstandes der Maske zündet.

2. Vorrichtung zur Modellierung oder Korrektur von optischen Linsen (5), insbesondere der Hornhaut des Auges, mit einem Impulslaser (1), dessen Strahlungsimpulse über optische Abbildungsmittel und Blenden derart auf die zu modellierende Oberfläche der Linse (5) gerichtet werden, daß konzentrische Kreisringflächen auf der Linsenoberfläche mit einer unterschiedlichen Zahl von Strahlungsimpulsen beaufschlagt und daher unterschiedlich tief abgetragen werden, wobei an der Linse (5) eine um eine linsenfeste, senkrecht auf der Linsenoberfläche stehende Achse drehbare Maske (4) gelagert ist, dadurch gekennzeichnet, daß die Maske (4) in radialer Richtung mehrere in Umfangsrichtung und konzentrisch zueinander verlaufende Ausnehmungen (9) aufweist, deren Länge in Umfangsrichtung verschieden ist, daß ein Antrieb (7) die Maske (4) schrittweise verdreht und daß eine Steuerung (8) für den Antrieb (7) und den Laser (1) vorgesehen ist, die den Laser (1) jeweils nach der Weiterdrehung der Maske (4) während des Stillstandes der Maske (4) zündet.

3. Vorrichtung nach Anspruch 2, dadurch gekennzeichnet, daß die Länge der Ausnehmungen (9) in der Maske (4) ein ganzzahliges Vielfaches der Schrittweite der Maskendrehung ist.

4. Vorrichtung nach Anspruch 2 oder 3, dadurch gekennzeichnet, daß die Maske (4) punktsymmetrisch bezüglich der Drehachse ausgebildet ist.

5. Vorrichtung nach einem der voranstehenden Ansprüche, dadurch gekennzeichnet, daß der Laserimpuls durch die optischen Abbildungsmittel auf eine balkenförmige Fläche abgebildet wird und daß durch weitere optische Elemente (2) die balkenförmige Strahlung senkrecht zur Längsausdehnung der balkenförmigen Fläche derart abgelenkt wird, daß die balkenförmige Strahlung die Maske (4) bei jedem Impuls vollständig überstreicht.

6. Vorrichtung nach einem der voranstehenden Ansprüche, dadurch gekennzeichnet, daß die Maske (4) an einer starr mit der Linse (5) verbundenen Halterung gelagert ist.

7. Vorrichtung nach Anspruch 6, dadurch gekennzeichnet, daß die Halterung mit Hilfe einer Unterdruckkammer an der Oberfläche der Linse (5) festgesaugt ist.

8. Vorrichtung nach Anspruch 6 oder 7, dadurch gekennzeichnet, daß die Maske (4) an der Halterung mittels eines Kugellagers gelagert ist.

9. Vorrichtung nach einem der voranstehenden Ansprüche, dadurch gekennzeichnet, daß die Umfangslänge der Ausnehmungen (9) im radial außen liegenden Bereich der Maske (4) von innen nach außen abnimmt.

10. Vorrichtung nach Anspruch 9, dadurch gekennzeichnet, daß die Umfangslänge der äußersten Ausnehmung (9) eine oder maximal einige wenige Schrittweiten des Maskenantriebs (7) beträgt.

## Claims

1. A method of shaping or correcting optical lenses with a pulsed laser whose radiation pulses are, via optical imaging means and stops, directed in such a manner onto the lens surface to be shaped that concentric annular areas of the lens surface are struck by a differing number of radiation pulses and are therefore removed to a differing depth, the lens surface to be treated being partially covered by means of a mask rotatable about an axis which is perpendicular to the lens surface and fast with the lens, characterised in that a mask is used which has in the radial direction a plurality of openings which run in the circumferential direction and concentrically to one another and which vary in length in the circumferential direction, in that the mask is rotated in a stepwise manner, and in that after each further rotation of the mask, the laser is activated whilst the mask is at a standstill.

2. A device for shaping or collecting optical lenses (5), in particular the cornea of the eye, with a pulsed laser (1) whose radiation pulses are, via optical imaging means and stops, directed in such a manner onto the lens (5) surface to be shaped that concentric annular areas of the lens surface are struck by a differing number of radiation pulses and are therefore removed to a differing depth, a mask (4), rotatable about an axis perpendicular to the lens surface and fast with the lens, being mounted on the lens (5), characterised in that the mask (4) has in the radial direction a plurality of openings (9) which run in the circumferential direction and concentrically to one another and which vary in length in the circumferential direction, in that the mask (4) is rotated in a stepwise manner by means of a drive (7), and in that a control device (8) for the drive (7) and the laser (1) is provided which after each further rotation of the mask (4) activates the laser (1) whilst the mask (4) is at a standstill.

3. A device in accordance with Claim 2, characterised in that the length of the openings (9) in the mask (4) is an integral multiple of the step width of the mask rotation.

4. A device in accordance with Claim 2 or 3, characterised in that the mask (4) is point-symmetric in relation to the rotational axis.

5. A device in accordance with any one of the preceding Claims, characterised in that the laser pulse is imaged onto a bar-shaped area through the optical imaging means, and in that the bar-shaped radiation is, through additional optical elements (2), deflected perpendicular to the longitudinal extension of the bar-shaped area in such a manner that the bar-shaped radiation sweeps over all of the mask (4) with each pulse.

6. A device in accordance with any one of the preceding Claims, characterised in that the mask (4) is mounted on a holding device rigidly connected to the lens (5).

7. A device in accordance with Claim 6, characterised in that the holding device is made fast on the surface of the lens (5) by means of suction with the aid of a low-pressure chamber.

8. A device in accordance with Claim 6 or 7, characterised in that the mask (4) is mounted on the holding device by means of a ball bearing.

9. A device in accordance with any one of the preceding Claims, characterised in that the circumferential length of the openings (9) in the radially outer region of the mask (4) decreases from the interior to the exterior.

10. A device in accordance with Claim 9, characterised in that the circumferential length of the outermost opening (9) amounts to one or at maximum a few step widths of the mask drive (7).

## Revendications

1. Procédé pour modeler ou corriger des lentilles optiques avec un laser à impulsions, dont les impulsions de rayonnement sont dirigées, via des moyens d'imagerie optique et des diaphragmes, de telle manière sur la surface de la lentille à modeler que des surfaces annulaires concentriques sur la surface de la lentille sont attaquées par un nombre différent d'impulsions de rayonnement, et font par conséquent l'objet d'un enlèvement à des profondeurs différentes, la surface de lentille à traiter étant recouverte partiellement au moyen d'un masque rotatif qui tourne autour d'un axe fixe par rapport à la lentille et perpendiculaire sur la surface de la lentille,
caractérisé en ce que l'on utilise un masque qui présente en direction radiale plusieurs évidements en direction périphérique et concentriques les uns par rapport aux autres, dont les longueurs sont différentes en direction circonférentielle, en ce qu'on fait tourner le masque pas à pas, et en ce qu'on déclenche le laser pendant l'arrêt du masque à chaque fois après nouvelle rotation du masque.

2. Appareil pour modeler ou corriger des lentilles optiques (5), en particulier la cornée oculaire, au moyen d'un laser à impulsions (1) dont les impulsions de rayonnement sont dirigées, via des moyens d'imagerie optique et des diaphragmes, sur la surface de la lentille à modeler (5) de telle manière que des surfaces annulaires concentriques sur la surface de la lentille sont attaquées par un nombre différent d'impulsions de rayonnement, et font par conséquent l'objet d'un enlèvement à des profondeurs différentes, un masque (4) qui tourne autour d'un axe fixe par rapport à la lentille et perpendiculaire à la surface de la lentille étant monté sur la lentille (5),
caractérisé en ce que le masque (4) présente en direction radiale plusieurs évidements (9) qui s'étendent en direction circonférentielle et concentriques les uns par rapport aux autres, dont les longueurs sont différentes en direction circonférentielle, en ce qu'un entraînement (7) fait tourner pas à pas le masque (4), et en ce qu'il est prévu une commande (8) pour l'entraînement (7) et le laser (1), qui déclenche le laser (1) pendant l'arrêt du masque (4) à chaque fois après une nouvelle rotation du masque (4).

3. Appareil selon la revendication 2, caractérisé en ce que la longueur des évidements (9) dans le masque (4) est un multiple entier du pas de la rotation du masque.

4. Appareil selon l'une ou l'autre des revendications 2 et 3, caractérisé en ce que le masque (4) est réalisé à symétrie ponctuelle par rapport à l'axe de rotation.

5. Appareil selon l'une quelconque des revendications précédentes, caractérisé en ce que l'impulsion laser est mise en forme d'une tache en forme de barrette à l'aide de moyens d'imagerie optique, et en ce que le rayonnement en forme de barrette est dévié par d'autres éléments optiques (2) perpendiculairement à l'extension longitudinale de la tache en forme de barrette, de telle manière que le rayonnement en forme de barrette balaye entièrement le masque (4) à chaque impulsion.

6. Appareil selon l'une quelconque des revendications précédentes, caractérisé en ce que le masque (4) est monté sur une monture rigidement reliée à la lentille (5).

7. Appareil selon la revendication 6, caractérisé en ce que la monture est fixement attachée par aspiration sur la surface de la lentille (5) à l'aide d'une chambre à dépression.

8. Appareil selon l'une ou l'autre des revendications 6 et 7, caractérisé en ce que le masque (7) est monté sur la monture au moyen d'un palier à billes.

9. Appareil selon l'une quelconque des revendications précédentes, caractérisé en ce que la longueur circonférentielle des évidements (9) diminue de l'intérieur vers l'extérieur dans la région du masque (4) située radialement à l'extérieur.

10. Appareil selon la revendication 9, caractérisé en ce que la longueur périphérique de l'évidement (9) situé le plus à l'extérieur s'élève à un ou au maximum à quelques pas de l'entraînement du masque (7).
